(19) **Europäisches Patentamt / European Patent Office / Office européen des brevets**

(11) **EP 4 640 047 A1**

(12) **EUROPEAN PATENT APPLICATION**
published in accordance with Art. 153(4) EPC

(43) Date of publication:
**29.10.2025 Bulletin 2025/44**

(21) Application number: **23906523.8**

(22) Date of filing: **10.11.2023**

(51) International Patent Classification (IPC):
*A01K 29/00* (2006.01)    *A61B 5/11* (2006.01)
*A61B 5/113* (2006.01)    *G01G 17/08* (2006.01)

(52) Cooperative Patent Classification (CPC):
**A01K 29/00; A61B 5/11; A61B 5/113; G01G 17/08**

(86) International application number:
**PCT/JP2023/040529**

(87) International publication number:
**WO 2024/135152 (27.06.2024 Gazette 2024/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.12.2022 JP 2022205073**

(71) Applicants:
• **Minebea Mitsumi Inc.**
  **Kitasaku-gun, Nagano 3890293 (JP)**
• **The University of Tokyo**
  **Bunkyo-ku, Tokyo 113-8654 (JP)**

(72) Inventors:
• **SEKIZAWA, Shinichi**
  **Tokyo 113-8654 (JP)**
• **SATO, Satoshi**
  **Kitasaku-gun, Nagano 389-0293 (JP)**
• **IIDA, Norihito**
  **Kitasaku-gun, Nagano 389-0293 (JP)**
• **TANAKA, Manabu**
  **Kitasaku-gun, Nagano 389-0293 (JP)**
• **NISHIMURA, Toshiaki**
  **Kitasaku-gun, Nagano 389-0293 (JP)**

(74) Representative: **Winter, Brandl - Partnerschaft mbB**
**Alois-Steinecker-Straße 22**
**85354 Freising (DE)**

(54) **HORSE STALL SYSTEM**

(57) A stall system (100) includes a load detector (10) installed at a floor surface of a stall and configured to detect a load of a target horse (OH), and a biological information generation unit (21) configured to generate, based on the load, biological information of the target horse.

FIG. 1

EP 4 640 047 A1

**Description**

Technical Field

The present invention relates to a stall system.

Background Art

Horses are used not only as racehorses but also in sports such as horse riding, farming, and the like. Patent Document 1 discloses an information providing device as a device for recognizing a state of a horse using a sensor. The information providing device of Patent Document 1 includes an acquisition unit acquiring biological information of a horse scheduled to run obtained by a vital sensor, and an output control unit outputting information including the biological information acquired by the acquisition unit to another device.

In addition, Patent Document 2 discloses a horse body weight measuring device including a measuring platform and a load sensor.

Citation List

Patent Literature

Patent Document 1: JP 2019-109631 A
Patent Document 2: JP 2011-52994 A

Summary of Invention

Technical Problem

Here, in the information providing device of Patent Document 1, it is necessary to attach the vital sensor to the horse in order to acquire the biological information of the horse. On the other hand, the horse body weight measuring device of Patent Document 2 can only measure a horse body weight.

In view of the above, an object of the present invention is to provide a stall system capable of acquiring biological information of a horse in a stall.

Solution to Problem

According to a first aspect of the present invention, a stall system includes a load detector installed at a floor surface of a stall and configured to detect a load of a target horse, and a biological information generation unit configured to generate, based on the load, biological information of the target horse.

Advantageous Effects of Invention

According to the stall system of the present invention, the biological information of the horse can be obtained in the stall.

Brief Description of Drawings

FIG. 1 is a block diagram illustrating a configuration of a stall system according to the present invention.
FIG. 2(a) is a side view of a load detector. FIG. 2(b) is a plan view of the load detector.
FIG. 3 is a flowchart illustrating a method of estimating a state of a target horse using the stall system.

Description of Embodiments

Embodiments

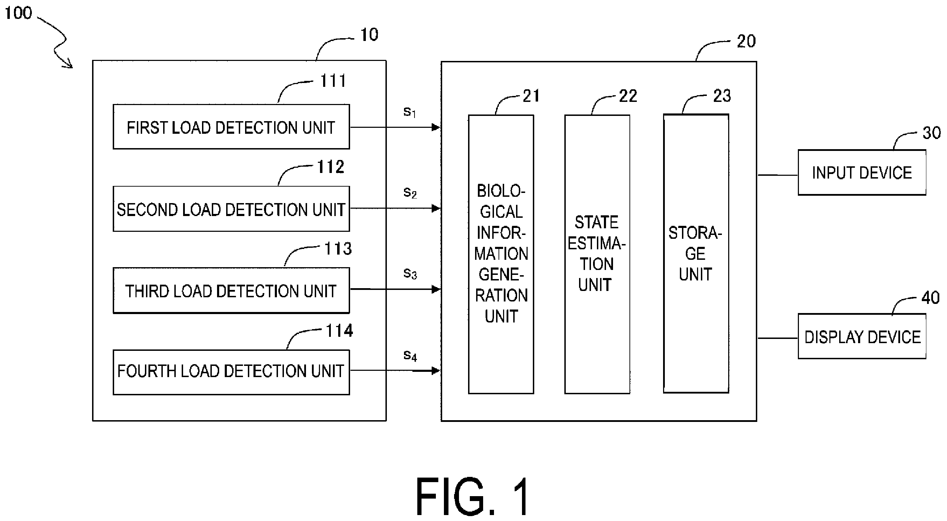

A stall system 100 of an embodiment of the present invention will be described with reference to FIG. 1 to FIG. 3.

In the stall system of the present embodiment, a load detector is disposed at a floor of a stall, and a load detected by the load detector is analyzed, so that biological information of a horse in the stall is generated. The stall system of the present embodiment estimates a state of the horse based on the generated biological information. Here, the stall is a space for the horse to spend a lot of time in a day, and one stall is prepared for each horse in most cases. For example, a plurality of stalls are arranged side by side in a stable. The stall includes, for example, a fixed structure such as a wall or a fence provided at a pair of long sides and one short side of a rectangular floor, and a movable body such as a door or a horse stop bar (bolt) provided at one short side of the rectangular floor. The area of the stall is, for example, about 2 m$^2$ to 4 m$^2$.

As illustrated in FIG. 1, the stall system 100 mainly includes a load detector 10, a control device 20, an input device 30, and a display device 40.

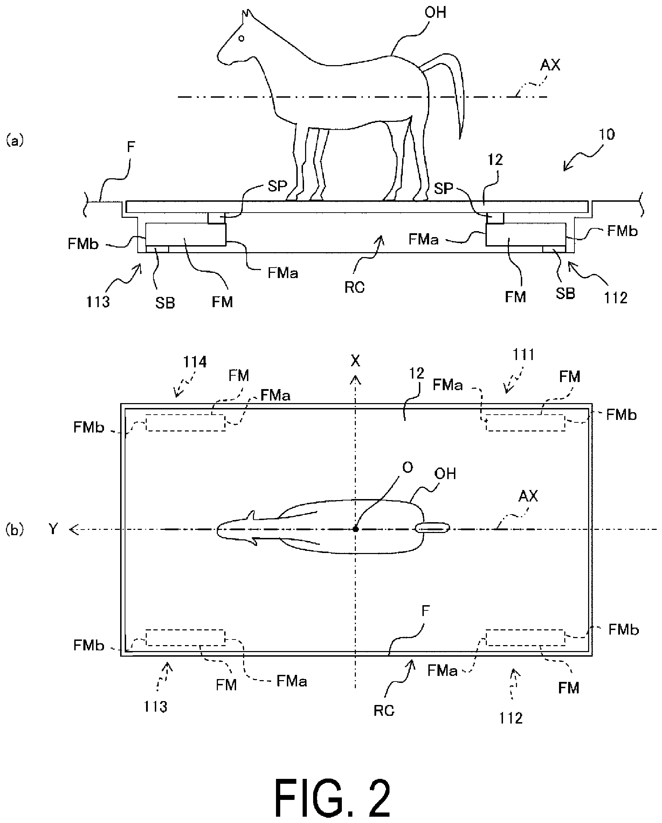

The load detector 10 is installed at a floor F (FIG. 2(a)) of the stall and detects a load of a horse being a subject (hereinafter referred to as a "target horse OH"). As illustrated in FIG. 2(a) and FIG. 2(b), the load detector 10 includes a first load detection unit 111, a second load detection unit 112, a third load detection unit 113, and a fourth load detection unit 114, and a rectangular placement plate 12 supported by these load detection units.

In the following description, for the sake of convenience, a short-side direction and a long-side direction of the placement plate 12 are referred to as an X direction and a Y direction, respectively (FIG. 2(b)), and a center of the placement plate 12 is referred to as an origin O. For each of the X direction and the Y direction, a positive side and a negative side are defined as illustrated in FIG. 2(a) and FIG. 2(b).

Each of the first load detection unit 111, the second load detection unit 112, the third load detection unit 113, and the fourth load detection unit 114 detects a load of the target horse OH. Since the first load detection unit 111 to the fourth load detection unit 114 have the same structure, the structure of the second load detection unit 112 will be representatively described here.

As illustrated in FIG. 2(a), the second load detection unit 112 includes a support base SB, a flexure element FM being an elongated member, and a spacer SP. The support base SB is disposed at a bottom surface of a recessed part RC having a rectangular shape in plan view (having substantially the same shape as the placement plate 12) and provided at the floor F of the stall, and supports the flexure element FM in a cantilever manner. A free end FMa (an end part opposite to a fixed end FMb fixed to the support base SB) of the flexure element FM is fixed to a bottom surface of the placement plate 12 via the spacer SP. A strain gauge (not illustrated) is attached to the flexure element FM, and the flexure element FM and the strain gauge constitute a load cell.

As illustrated in FIG. 2(b), the first load detection unit 111 is disposed at the positive side in the X direction and the negative side in the Y direction such that a longitudinal direction of the flexure element FM coincides with the Y direction. The free end FMa of the flexure element FM is located at the positive side in the Y direction relative to the fixed end FMb of the flexure element FM. The second load detection unit 112 is disposed at the negative side in the X direction and the negative side in the Y direction such that a longitudinal direction of the flexure element FM coincides with the Y direction. The free end FMa of the flexure element FM is located at the positive side in the Y direction relative to the fixed end FMb of the flexure element FM.

The third load detection unit 113 is disposed at the negative side in the X direction and the positive side in the Y direction such that a longitudinal direction of the flexure element FM coincides with the Y direction. The free end FMa of the flexure element FM is located at the negative side in the Y direction relative to the fixed end FMb of the flexure element FM. The fourth load detection unit 114 is disposed at the positive side in the X direction and the positive side in the Y direction such that a longitudinal direction of the flexure element FM coincides with the Y direction. The free end FMa of the flexure element FM is located at the negative side in the Y direction relative to the fixed end FMb of the flexure element FM.

The placement plate 12 functions as a weighing platform of the load detector 10. In the present embodiment, the placement plate 12 is a flat plate rectangular in plan view and made of metal (as an example, stainless steel, steel, or the like).

The placement plate 12 is supported by the first load detection unit 111 to the fourth load detection unit 114 in the vicinities of four corners of the placement plate 12. In the present embodiment, the placement plate 12 is disposed inside the recessed part RC with a slight gap between the placement plate 12 and a wall surface defining the recessed part RC. An upper surface of the placement plate 12 may be flush with an upper surface of the floor F of the stall.

In the present embodiment, the floor F of the stall has a rectangular shape in plan view, and a long-side direction of the floor F coincides with the Y direction. That is, the long-side direction of the floor F, the long-side direction of the placement plate 12, and a long-side direction of the recessed part RC coincide with each other, and a short-side direction of the floor F, the short-side direction of the placement plate 12, and a short-side direction of the recessed part RC coincide with each other. An entrance/exit of the stall is at the positive side in the Y direction. The entrance/exit is provided with a door. However, the arrangement of the placement plate 12, the recessed part RC, and the like relative to the stall is not limited to this arrangement.

The control device 20 acquires various types of information related to the target horse OH based on a load of the target horse OH detected by the load detector 10. The control device 20 is connected to the first load detection unit 111 to the fourth load detection unit 114 of the load detector 10 in a wired or wireless manner.

The control device 20 is a dedicated or general-purpose computer, and internally includes a biological information generation unit 21, a state estimation unit 22, and a storage unit 23.

The biological information generation unit 21 generates biological information of the target horse OH based on a load of the target horse OH detected by the load detector 10. The generated biological information includes, for example, information related to respiration of the target horse OH, information related to a heartbeat of the target horse OH, information related to a center of gravity of the target horse OH, information related to a load (weight) of the target horse OH, and the like. The biological information generation unit 21 will be described in detail below.

The state estimation unit 22 estimates a state of the target horse OH based on biological information of the target horse OH generated by the biological information generation unit 21. The estimated state of the target horse OH includes the presence or absence of an injury or a sign of injury, whether or not the target horse OH is in heat or rut, the presence or absence of a disease, a posture, and the like. The state estimation unit 22 will be described in detail below.

The storage unit 23 stores data necessary for an operation of the control device 20, data generated through the operation of the control device 20, and the like. The storage unit 23 may be, for example, a hard disk, a nonvolatile memory, or the like.

Note that the control device 20 can be configured of, for example, a central processing unit (CPU), a graphics processing unit (GPU), or the like. Various functional blocks such as the biological information generation unit 21 and the state estimation unit 22 included in the control device 20 may be appropriately implemented by the control device 20 as a CPU reading and executing program data stored in the storage unit 23.

The input device 30 is a user interface for performing a predetermined input to the control device 20. The input device 30 is connected to the control device 20 in a wired or wireless manner. The input device 30 may be, for example, a touch panel, a keyboard, a mouse, or the like.

The display device 40 is a user interface displaying information to a user of the stall system 100. The display device 40 is connected to the control device 20 in a wired or wireless manner.

The display device 40 may be, for example, a visual display device such as a display visually displaying information. Further, the visual display device may be integrated with a touch panel of the input device 30. The display device 40 may include an audio output device in addition to the visual display device or instead of the visual display device. The audio output device presents information through audio to the user of the stall system 100.

At least two of the control device 20, the input device 30, and the display device 40 may be integrally configured.

A method of estimating a state of the target horse OH in the stall using the stall system 100 according to the present embodiment will be described.

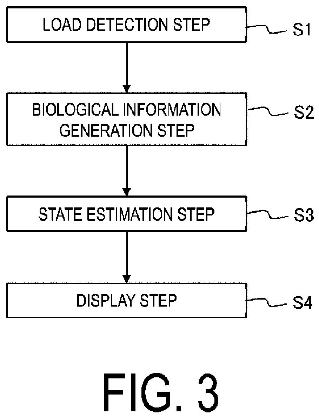

As illustrated in a flowchart of FIG. 3, the estimation of the state of the target horse OH using the stall system 100 includes a load detection step S1 of detecting a load of the target horse OH, a biological information generation step S2 of generating biological information of the target horse OH based on the detected load of the target horse OH, a state estimation step S3 of estimating a state of the target horse OH based on the generated biological information, and a display step S4 of displaying the estimated state of the target horse OH on the display device 40.

Load Detection Step S1

In the load detection step S1, the load detector 10 is used to detect a load of the target horse OH at the placement plate 12. The load of the target horse OH at the placement plate 12 is applied in a distributed manner to the first load detection unit 111 to the fourth load detection unit 114 disposed at the four corners of the placement plate 12 and is detected by these load detection units in a distributed manner.

The first load detection unit 111 to the fourth load detection unit 114 each detect a load (load change) and output the load as an analog signal. The output analog signal is converted into a digital signal by an A/D conversion unit (not illustrated) and is input to the control device 20. Hereinafter, the digital signals acquired by performing A/D conversion on the analog signals output from the first load detection unit 111, the second load detection unit 112, the third load detection unit 113, and the fourth load detection unit 114 are referred to as load signals $s_1$, $s_2$, $s_3$, and $s_4$, respectively. A sampling period of the A/D conversion is freely determined, but may be set to 5 milliseconds as an example.

Biological Information Generation Step S2

In the biological information generation step S2, the biological information generation unit 21 generates (calculates, acquires) biological information of the target horse OH based on the load signals $s_1$, $s_2$, $s_3$, and $s_4$. Examples of the generated biological information and a method of generating the biological information are as follows.

(1) Partial Load

A load of the target horse OH indicated by each of the load signals $s_1$, $s_2$, $s_3$, and $s_4$ (i.e., part of a load of the target horse OH (partial load)) is also one type of biological information. Hereinafter, the partial loads of the target horse OH based on the load signals $s_1$, $s_2$, $s_3$, and $s_4$ are referred to as partial loads $W_1$, $W_2$, $W_3$, and $W_4$, respectively.

(2) Total Load (Weight, Horse Body Weight)

A total load (weight, horse body weight) of the target horse OH is calculated by summing up the partial loads $W_1$, $W_2$, $W_3$, and $W_4$ of the target horse OH indicated by the load signals $s_1$, $s_2$, $s_3$, and $s_4$. Hereinafter, this load is referred to as a total load W.

(3) Position of Center of Gravity

A position of a center of gravity G of the target horse OH is a position of the center of gravity G of the target horse OH at the placement plate 12, that is, a position of the center of gravity G of the target horse OH in XY coordinates of FIG. 2(b) (hereinafter, simply referred to as XY coordinates). The position of the center of gravity G is calculated by following Equation 1 and Equation 2. In the equations, the position of the center of gravity G in the XY coordinates is referred to as (x, y), and coordinates of the free ends of the first load detection unit 111, the second load detection unit 112, the third load detection unit 113, and the fourth load detection unit 114 in the XY coordinates are referred to as $(X_1, Y_1)$, $(X_2, Y_2)$, $(X_3, Y_3)$, and $(X_4, Y_4)$, respectively.

[Mathematical Expression 1]

$$(\text{Equation 1})$$

$$x = \frac{X_1 \times W_1 + X_2 \times W_2 + X_3 \times W_3 + X_4 \times W_4}{W_1 + W_2 + W_3 + W_4}$$

[Mathematical Expression 2]

$$(\text{Equation 2})$$

$$y = \frac{Y_1 \times W_1 + Y_2 \times W_2 + Y_3 \times W_3 + Y_4 \times W_4}{W_1 + W_2 + W_3 + W_4}$$

(4) Center of Gravity Trajectory

A center of gravity trajectory GT, that is, a trajectory of a temporal variation in the position of the center of gravity G is also one type of biological information. The center of gravity trajectory GT is generated based on the position of the center of gravity G of the target horse OH calculated at a predetermined period.

(5) Deviation of Center of Gravity

Information indicating that the position of the center of gravity G deviates is also one type of biological information. Specifically, for example, the biological information generation unit 21 can determine that the position of the center of gravity G deviates based on the fact that an average position of the center of gravity G in a predetermined period deviates to one side relative to a past average position.

(6) Respiration Information

Respiration information of the target horse OH includes a respiration rate, a depth of respiration, and the like of the target horse OH. The respiration information of the target horse OH is obtained as follows based on the partial loads $W_1$, $W_2$, $W_3$, and $W_4$ of the target horse OH, the total load W of the target horse OH, the position of the center of gravity G, the center of gravity trajectory GT, and the like.

As an example of a method of generating the respiration information, a method of generating respiration information of a horse based on movement of the center of gravity of the horse in response to respiration of the horse will be described.

In the body of the horse, the lungs are present in a region at a head side (rostral side, front side) of the trunk, and organs such as the liver are present at a tail side (rear side) of the lungs via the diaphragm.

Here, when the horse inhales, the diaphragm moves toward the tail, and the liver also moves toward the tail. Thus, the center of gravity of the horse moves toward the tail. On the other hand, when the horse exhales, the diaphragm moves toward the head, and the liver also moves toward the head. Thus, the center of gravity of the horse moves toward the head. Therefore, respiration causes the center of gravity of the horse to oscillate along a direction of a body axis of the horse extending between the head side and the tail side (i.e., draws a simple harmonic trajectory).

Therefore, for example, the biological information generation unit 21 generates respiration information of the target horse OH based on the trajectory of the center of gravity G oscillating along a body axis AX (FIG. 2(a)) of the target horse OH. This trajectory is included in the center of gravity trajectory GT of the target horse OH. Specifically, for example, the biological information generation unit 21 calculates the respiration rate of the target horse OH based on the oscillation period of the center of gravity G along the body axis AX, and generates information related to the depth of respiration of the target horse OH based on the amplitude of the oscillation of the center of gravity G along the body axis AX.

According to findings of the inventors of the present invention, the moving speed of the center of gravity of a horse in response to exhalation is generally higher than the moving speed of the center of gravity in response to inhalation. Therefore, it is possible to determine whether a horse is exhaling or inhaling based on the movement speed of the center of gravity of the horse in the body axis direction of the horse.

As another example of the method of generating respiration information, a method of generating respiration information of a horse based on the partial loads of the horse will be described.

The respiration rate of a horse (adult horse) at rest is generally about 8 to 20 times per minute. That is, the frequency of respiration of a horse is generally about 0.13 Hz to 0.33 Hz.

Here, since the center of gravity G moves in response to respiration of the target horse OH, each of the partial loads $W_1$, $W_2$, $W_3$, and $W_4$ also slightly varies in response to respiration of the target horse OH. Therefore, for example, the biological information generation unit 21 performs frequency analysis (as an example, fast Fourier transformation) on a waveform indicating a temporal variation in any one of the partial loads $W_1$, $W_2$, $W_3$, and $W_4$, and generates respiration information of the target horse OH based on a value of a peak frequency appearing in a predetermined band (as an example, a band from 0.13 Hz to 0.33 Hz) corresponding to respiration of the horse. Specifically, for example, the biological information generation unit 21 calculates the respiration rate of the target horse OH based on the value of the specified peak frequency. As an example, the respiration rate of the target horse OH per minute can be calculated by multiplying the value of the specified peak frequency by 60.

As another example of the method of generating respiration information, a method of generating respiration information of a horse based on the total load of the horse (horse body weight) will be described.

According to findings of the inventors of the present invention, when a horse respires, a mechanical vector is generated in the vertical direction due to expansion and contraction of the lungs of the horse and may affect a measurement value of the total load (horse body weight) of the horse. Therefore, for example, it is possible to generate respiration information of the horse based on measurement of a fluctuation (that is, temporal variation) in a measurement value of the total load of the horse in response to respiration of the horse.

Specifically, for example, the biological information generation unit 21 performs frequency analysis (as an example, fast Fourier transformation) on a waveform indicating a temporal variation in the total load W of the target horse OH, and calculates a value of a peak frequency appearing in a predetermined band (as an example, a band from 0.13 Hz to 0.33 Hz) corresponding to respiration of the horse. Then, respiration information (for example, respiration rate) of the target horse OH is generated based on the obtained value of the peak frequency.

In addition, it is also possible to acquire more detailed respiration information by considering a habitual sitting posture of the target horse OH in the stall.

(7) Heartbeat Information

Heartbeat information of the target horse OH includes a heart rate, a depth of heartbeat, and the like of the target horse OH. The heartbeat information of the target horse OH is obtained as follows based on the partial loads $W_1$, $W_2$, $W_3$,

and $W_4$ of the target horse OH, the total load W of the target horse OH, the position of the center of gravity G, the center of gravity trajectory GT, and the like.

As an example of a method of generating the heartbeat information, a method of generating heartbeat information of a horse based on the partial loads of the horse will be described.

The heart rate of a horse (adult horse) at rest is generally about 20 to 40 times per minute and is about four times as frequent as the respiration rate. That is, the frequency of the heartbeat of a horse is generally about 0.33 to 0.67 Hz. Therefore, for example, a component having a frequency higher than the respiration frequency (as an example, a component having a frequency equal to or higher than 0.1 Hz) is extracted by filtering or the like from a waveform indicating a temporal variation in any one of the partial loads $W_1$, $W_2$, $W_3$, and $W_4$. Accordingly, a respiration waveform having a frequency of about 0.13 Hz to 0.33 Hz and a heartbeat waveform having a frequency of about 0.33 Hz to 0.67 Hz and superimposed on the respiration waveform as a notch (a small wave having a V-shape, a U-shape, or the like) are obtained. The biological information generation unit 21 generates heartbeat information of the target horse OH based on the heartbeat waveform.

Specifically, for example, the biological information generation unit 21 calculates the heart rate of the target horse OH based on the period of appearance of the notch of the heartbeat waveform, and generates information related to the magnitude of the heartbeat of the target horse OH based on the amplitude of the heartbeat waveform. As an example, the heart rate per minute can be calculated by dividing 60 [seconds] by the period [seconds] of appearance of the notch.

Note that a component having a frequency higher than the heartbeat frequency (as an example, a component having a frequency equal to or higher than 0.33 Hz) may be extracted by filtering or the like from a waveform indicating a temporal variation in any one of the partial loads $W_1$, $W_2$, $W_3$, and $W_4$ (that is, the heartbeat waveform may be extracted without extracting the respiration waveform). In addition, the biological information generation unit 21 may perform frequency analysis on a waveform indicating a temporal variation in any one of the partial loads $W_1$, $W_2$, $W_3$, and $W_4$, and generate heartbeat information of the target horse OH based on a value of a peak frequency appearing in a predetermined band (for example, a band from 0.33 Hz to 0.67 Hz) corresponding to the heartbeat of the horse.

As another example of the method of generating heartbeat information, a method of generating heartbeat information of a horse based on the total load of the horse (horse body weight) will be described.

When the horse's heart pumps blood once, a mechanical vector is generated in the vertical direction due to the pumping action of the heart and may affect a measurement value of the total load of the horse (horse body weight). Therefore, for example, it is possible to generate heartbeat information of the horse based on measurement of a fluctuation (that is, temporal variation) in a measurement value of the total load of the horse in response to the heartbeat of the horse.

Specifically, for example, the biological information generation unit 21 performs frequency analysis (as an example, fast Fourier transformation) on a waveform indicating a temporal variation in the total load W of the target horse OH, and calculates a value of a peak frequency appearing in a predetermined band (as an example, a band from 0.33 Hz to 0.67 Hz) corresponding to the heartbeat of the horse. Then, heartbeat information (for example, heart rate) of the target horse OH is generated based on the obtained value of the peak frequency. For example, the heart rate per minute can be calculated by multiplying the obtained value of the peak frequency by 60.

In addition, when a horse stimulates an activity such as shaking of a skin muscle or skeletal muscle in order to adapt to an external temperature environment, a specific weight variation caused by the shaking of the skin muscle and skeletal muscle is observed. Therefore, for example, the biological information generation unit 21 may generate information indicating that the target horse OH attempts to adapt to the external temperature environment based on the fact that the total load W of the target horse OH shows the specific variation.

State Estimation Step S3

In the state estimation step S3, the state estimation unit 22 estimates a state of the target horse OH based on the biological information generated in the biological information generation step S2. An example of the estimated state and a method of estimating the state are as follows.

(1) Presence or Absence of Injury or Sign of Injury

In the case of a heavy quadruped animal such as a horse, the body weight loads applied to the four limbs are desirably uniform, and, in the case of a horse having no injury or no sign of injury, the body weight loads applied to the four limbs are substantially uniform. Therefore, by comparing the compression densities of the legs and the vicinities of the legs and detecting uniformity and/or non-uniformity of the body weight loads applied to the four limbs, it is possible to detect that there is an injury or a sign of an injury in the legs.

Specifically, for example, the state estimation unit 22 estimates whether or not the target horse OH has an injury or a sign of an injury based on an imbalance degree of the partial loads $W_1$, $W_2$, $W_3$, and $W_4$ (information related to the loads applied to the respective legs of the target horse OH). In this case, when it is determined that the partial loads $W_1$, $W_2$, $W_3$,

and $W_4$ are uniform or substantially uniform, the state estimation unit 22 estimates that the target horse OH has no injury or no sign of injury. When it is determined that the partial loads $W_1$, $W_2$, $W_3$, and $W_4$ are not uniform, the state estimation unit 22 estimates that the target horse OH has an injury or a sign of injury. A threshold value for determining whether or not the partial loads are uniform can be set as appropriate.

Note that values of the partial loads $W_1$, $W_2$, $W_3$, and $W_4$ are affected by the position of the target horse OH at the placement plate 12. Therefore, when it is determined whether or not the partial loads $W_1$, $W_2$, $W_3$, and $W_4$ are uniform, the values of the partial loads $W_1$, $W_2$, $W_3$, and $W_4$ can be adjusted based on the position of the center of gravity G of the target horse OH. Specifically, for example, when the center of gravity G of the target horse OH is located at the positive side in the X direction, determination can be performed after the values of the partial loads $W_1$ and $W_4$ are decreased and the values of the partial loads $W_2$ and $W_3$ are increased. In this way, by performing the determination after adjusting the values of the partial loads $W_1$, $W_2$, $W_3$, and $W_4$ based on the position of the center of gravity G of the target horse OH, it is possible to suppress influence of variations in the values of the partial loads $W_1$, $W_2$, $W_3$, and $W_4$ affected by the position of the target horse OH at the placement plate 12. Accordingly, it is possible to accurately determine whether or not the partial loads $W_1$, $W_2$, $W_3$, and $W_4$ are uniform regardless of the position of the target horse OH at the placement plate 12.

In addition, when a horse has an injury or a sign of injury at a leg part, the horse tends to move the center of gravity to a half body (right half body or left half body) at a side opposite to the leg having the injury or the sign of injury in order to favor the leg. Therefore, the state estimation unit 22 can estimate whether or not the target horse OH has an injury or a sign of injury based on a deviation degree of the center of gravity G. Specifically, for example, when the average position of the center of gravity G of the target horse OH in a predetermined period deviates from a predetermined reference position (for example, an average center of gravity position of the target horse OH calculated based on past measurements) by more than a predetermined threshold value, the state estimation unit 22 can determine that the target horse OH has an injury or a sign of injury.

In addition, according to findings of the inventors of the present invention, in a state of moving the center of gravity to a half body (right half body or left half body) at a side opposite to a leg having an injury or a sign of injury in order to favor the leg, the horse is unstable, and a fluctuation (temporal variation) in a detection value of the total load W becomes large. Therefore, for example, when the temporal variation in the total load W of the target horse OH (for example, the standard deviation of the temporal variation in the total load W) is equal to or greater than a predetermined threshold value, the state estimation unit 22 can estimate that the target horse OH has an injury or a sign of injury.

(2) Whether Horse Is in Heat or Rut

Between winter and spring being a breeding season of horses, each horse is in heat or rut one to several times. A horse in heat or rut becomes restless and often changes a posture or moves within a stall. Therefore, for example, the state determination unit 22 can estimate that the target horse OH is in heat or rut based on the center of gravity trajectory GT of the target horse OH, a pattern indicated by the center of gravity trajectory GT, a periodicity of movement of the center of gravity G indicated by the center of gravity trajectory GT, and the like.

Specifically, for example, a table is stored in the storage unit 23. In the table, a predetermined pattern of the center of gravity trajectory GT (one type or a plurality of types) is associated with information indicating that the target horse OH is in heat or rut. Then, the state estimation unit 22 estimates that the target horse OH is in heat or rut based on the table and the center of gravity trajectory GT of the target horse OH received from the biological information generation unit 21. As an example, when the center of gravity trajectory GT received from the biological information generation unit 21 is the same as or similar to the predetermined pattern stored in the table, the state estimation unit 22 can estimate that the target horse OH is in heat or rut.

(3) Presence or Absence of Disease

For horses, colic (a general term for diseases with abdominal pain, mainly caused by the digestive tract) is a serious disease associated with vital prognosis. A horse with colic feels severe pain and may take an action such as standing up or jumping up. Therefore, for example, it is possible to estimate that the target horse OH has colic based on an instantaneous increase in the total load W or at least one of the partial loads $W_1$, $W_2$, $W_3$, or $W_4$ of the target horse OH caused when the target horse OH takes an action such as standing up or jumping up at the placement plate 12. Specifically, for example, the state estimation unit 22 can estimate that the target horse OH has colic when a variation range of the total load W or at least one of the partial loads $W_1$, $W_2$, $W_3$, or $W_4$ of the target horse OH in a predetermined period (for example, a short period suitable for determining the presence or absence of an instantaneous increase) exceeds a predetermined threshold value.

In addition, a horse having colic may be in a decubitus position (lateral position, spine position, or the like). In this case, the center of gravity trajectory of the horse becomes a trajectory moving instantaneously. This trajectory is greatly different from a normal trajectory. Therefore, for example, the state estimation unit 22 can estimate that the target horse OH has colic based on the fact that the center of gravity G of the target horse OH has moved beyond a predetermined distance in a predetermined period (for example, a short period suitable for determining an amount of instantaneous movement of the center of gravity).

(4) Posture

The state estimation unit 22 can estimate the posture of the target horse OH as the state of the target horse OH. Here, the posture of the target horse OH includes the orientation of the body axis AX of the target horse OH and/or the arrangement of the head part and/or the tail part of the target horse OH in the direction of the body axis AX.

As described above, the center of gravity of the horse oscillates along the body axis of the horse in response to respiration of the horse. Therefore, the state estimation unit 22 can estimate the extension direction of the body axis AX of the target horse OH based on the oscillation direction of the trajectory oscillating in response to respiration of the target horse OH. This trajectory is included in the center of gravity trajectory GT of the target horse OH.

Further, as described above, the moving speed of the center of gravity of the horse in response to exhalation of the horse is higher than the moving speed of the center of gravity of the horse in response to inhalation of the horse. Therefore, the state estimation unit 22 can estimate at which side of the oscillation direction of the trajectory the head part of the target horse OH is located and at which side of the oscillation direction of the trajectory the tail part of the target horse OH is located, based on the speed of the center of gravity G in the trajectory oscillating in response to respiration of the target horse OH. This trajectory is included in the center of gravity trajectory GT of the target horse OH. Specifically, for example, regarding the trajectory oscillating in response to respiration of the target horse OH, the state estimation unit 22 calculates the speed of the center of gravity G toward one side in the oscillation direction and the speed of the center of gravity G toward another side in the oscillation direction. Then, the trajectory of the center of gravity G having a low moving speed is regarded as a trajectory in response to inhalation, and it is estimated that the head part of the target horse OH is located at the start point side of the trajectory and the tail part of the target horse OH is located at the end point side of the trajectory.

In addition, according to findings of the inventors of the present invention, each horse normally has a preferred position (i.e., a fixed position) for spending time in a stall. When the horse has some kind of abnormality, the horse tends to spend time in a situation. In the situation, the horse is away from the preferred fixed position for normally spending time. Therefore, for example, by accumulating positional data of the target horse OH in the stall and using the data as background values, it is possible to estimate that the target horse OH has an abnormality when spending time in a situation. In the situation, the target horse OH is away from the fixed position. According to this estimation method, it is also possible to estimate the presence or absence of an abnormality at a part other than the leg parts (for example, an internal abnormality such as a disease).

Specifically, for example, an average position of the center of gravity G, an average distribution region of the center of gravity trajectory GT, and the like in a past predetermined period are stored in the storage unit 23. Then, the state estimation unit 22 can estimate that the target horse OH has an internal abnormality based on the fact that the center of gravity trajectory GT of the target horse OH becomes a peculiar trajectory different from a past center of gravity trajectory GT (for example, remaining at a position separated from the average distribution region by more than a predetermined threshold value for a period more than a predetermined threshold value).

Display Step S4

In the display step S4, the biological information generated in the biological information generation step S2, the state determined in the state determination step S3, and the like are displayed on the display device 40. Note that when the biological information of the target horse OH and/or the state of the target horse OH satisfies a predetermined condition, the display device 40 may issue a visual and/or audible alert.

The effects of the stall system 100 according to the present embodiment are summarized below.

According to the stall system 100 of the present embodiment, since the floor F of the stall is provided with the load detector 10, it is possible to acquire biological information of the target horse OH in the stall.

According to the stall system 100 of the present embodiment, it is possible to suitably estimate a state of the target horse OH using the load detector 10 installed at the floor F of the stall.

The stall system 100 of the present embodiment detects a load using the load detector 10 installed at the floor F of the stall, and acquires biological information of the target horse OH based on the load. Therefore, it is not necessary to attach a sensor to the target horse OH in order to acquire biological information, and stress imposed on the target horse OH is small.

In the stall system 100 of the present embodiment, the load detector 10 is installed in the stall and, in the stall, the target horse OH spends a lot of time. Thus, there is no trouble of guiding the target horse OH to a measuring device or the like in order to acquire biological information. In addition, since it is possible to continuously acquire information from the target horse OH over a long period of time, it is possible to generate biological information of the target horse OH with high accuracy. Then, it is possible to estimate a state of the target horse OH with high accuracy based on the biological information acquired with high accuracy.

Modifications

In the embodiment described above, the following modified aspects can also be used.

In the stall system 100 of the above-described embodiment, the number of the load detection units included in the load detector 10 is not limited to four. The load detector 10 can include three or less, or five or more load detection units.

In the stall system 100 according to the above-described embodiment, it is not essential to estimate a state of the target horse OH. That is, in the stall system 100, the state estimation unit 22 of the control device 20 is not an essential component. When the state of the target horse OH is not estimated, the stall system 100 executes the load detection step S 1 and the biological information generation step S2, and then executes the display step S4 without executing the state estimation step S3. The display step S4 may be omitted, and in this case, for example, biological information may be only collected and accumulated.

As long as the features of the present invention are maintained, the present invention is not limited to the embodiment described above, and other forms considered within the scope of the technical concept of the present invention are also included within the scope of the present invention.

Industrial Applicability

According to the stall system of the present invention, it is possible to generate biological information of a target horse and utilize the biological information for various treatments or the like in order to keep the target horse in a good state, for example.

Reference Signs List

10 Load detector, 111 First load detection unit, 112 Second load detection unit, 113 Third load detection unit, 114 Fourth load detection unit, 12 Placement plate, 20 Control device, 21 Biological information generation unit, 22 State estimation unit, 23 Storage unit, 30 Input device, 40 Display device, 100 Stall system, OH Target horse

**Claims**

1. A stall system comprising:

   a load detector installed at a floor surface of a stall and configured to detect a load of a target horse, and
   a biological information generation unit configured to generate, based on the load, biological information of the target horse.

2. The stall system according to claim 1, wherein
   the biological information includes information related to respiration of the target horse and/or information related to a heartbeat of the target horse.

3. The stall system according to claim 1 or 2, wherein
   the biological information includes information related to a center of gravity of the target horse.

4. The stall system according to any one of claims 1 to 3, comprising:
   a state estimation unit configured to estimate, based on the biological information, a state of the target horse.

5. The stall system according to claim 4, wherein

   the state of the target horse is whether or not the target horse has an injury or a sign of injury,
   the biological information includes information related to a load applied to an individual leg of the target horse, information related to a center of gravity of the target horse, and/or information indicating a total load of the target horse, and
   the state estimation unit estimates whether or not the target horse has an injury or a sign of injury, based on an imbalance degree of the load applied to the individual leg of the target horse, a deviation degree of the center of gravity of the target horse, and/or a temporal variation in the total load of the target horse.

6. The stall system according to claim 4 or 5, wherein

the state of the target horse is whether or not the target horse is in heat or rut,
the biological information includes information related to a trajectory of a center of gravity position of the target horse, and
the state estimation unit estimates whether or not the target horse is in heat or rut, based on at least one of the trajectory of the center of gravity position, a pattern indicated by the trajectory, or a periodicity of movement of the center of gravity position indicated by the trajectory.

7. The stall system according to any one of claims 4 to 6, wherein

the state of the target horse is whether or not the target horse has a disease,
the biological information includes at least one of information indicating a total load of the target horse, information related to a load applied to an individual leg of the target horse, or information related to a trajectory of a center of gravity position of the target horse, and
the state estimation unit estimates whether or not the target horse has a disease, based on a variation range of the total load or the load applied to the individual leg and/or a movement amount of the center of gravity position within a predetermined time.

8. The stall system according to any one of claims 4 to 7, wherein

the state of the target horse is a posture of the target horse,
the biological information includes information related to a trajectory of a center of gravity position of the target horse, and
the state estimation unit estimates, as the posture of the target horse, an orientation of a body axis of the target horse and/or an arrangement of a head part and/or a tail part of the target horse, based on the trajectory of the center of gravity position of the target horse.

**FIG. 1**

EP 4 640 047 A1

FIG. 2

LOAD DETECTION STEP — S1

↓

BIOLOGICAL INFORMATION GENERATION STEP — S2

↓

STATE ESTIMATION STEP — S3

↓

DISPLAY STEP — S4

# FIG. 3

## INTERNATIONAL SEARCH REPORT

| International application No. |
|---|
| **PCT/JP2023/040529** |

### A. CLASSIFICATION OF SUBJECT MATTER

*A01K 29/00*(2006.01)i; *A61B 5/11*(2006.01)i; *A61B 5/113*(2006.01)i; *G01G 17/08*(2006.01)i
FI: A01K29/00 A; A61B5/113; G01G17/08; A01K29/00 D; A61B5/11 100; A61B5/11 210

According to International Patent Classification (IPC) or to both national classification and IPC

### B. FIELDS SEARCHED

Minimum documentation searched (classification system followed by classification symbols)

A01K29/00; A61B5/11; A61B5/113; G01G17/08

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2011-52994 A (NAGAYAMA, Kazuo, AOKI, Satoshi) 17 March 2011 (2011-03-17) paragraphs [0016]-[0018], fig. 1 | 1 |
| A | | 2-8 |
| X | JP 4-307334 A (KUBOTA CORP) 29 October 1992 (1992-10-29) paragraphs [0008]-[0011] | 1, 3-4 |
| A | | 2, 5-8 |
| Y | JP 2016-176796 A (DAINIPPON PRINTING CO LTD) 06 October 2016 (2016-10-06) paragraphs [0014]-[0048] | 2, 4, 7 |
| Y | JP 2007-236251 A (TERUMO CORP) 20 September 2007 (2007-09-20) paragraphs [0006]-[0026] | 2, 4, 7 |
| A | JP 2016-96758 A (FUJITSU LTD) 30 May 2016 (2016-05-30) entire text, all drawings | 1-8 |

✓ Further documents are listed in the continuation of Box C.    ✓ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **17 January 2024** | **30 January 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**EP 4 640 047 A1**

| | International application No. |
|---|---|
| | **PCT/JP2023/040529** |

**C.    DOCUMENTS CONSIDERED TO BE RELEVANT**

| Category* | Citation of document, with indication, where appropriate, of the relevant passages | Relevant to claim No. |
|---|---|---|
| A | JP 58-162222 A (EICHIAARUEICHI IND ANDO TOREIDEINGU LTD) 26 September 1983 (1983-09-26)<br>entire text, all drawings | 1-8 |
| A | US 2016/0073614 A1 (LAMPE, Kyle Douglas) 17 March 2016 (2016-03-17)<br>entire text, all drawings | 1-8 |
| A | US 2021/0113155 A1 (BOEHRINGER INGELHEIM VETMEDICA GMBH) 22 April 2021 (2021-04-22)<br>entire text, all drawings | 1-8 |

Form PCT/ISA/210 (second sheet) (July 2022)

## INTERNATIONAL SEARCH REPORT
### Information on patent family members

International application No.

**PCT/JP2023/040529**

| Patent document cited in search report | | | Publication date (day/month/year) | Patent family member(s) | | | Publication date (day/month/year) |
|---|---|---|---|---|---|---|---|
| JP | 2011-52994 | A | 17 March 2011 | (Family: none) | | | |
| JP | 4-307334 | A | 29 October 1992 | (Family: none) | | | |
| JP | 2016-176796 | A | 06 October 2016 | (Family: none) | | | |
| JP | 2007-236251 | A | 20 September 2007 | (Family: none) | | | |
| JP | 2016-96758 | A | 30 May 2016 | US entire text, all drawings | 2016/0135716 | A1 | |
| JP | 58-162222 | A | 26 September 1983 | EP entire text, all drawings EP | 83848 83847 | A1 A2 | |
| US | 2016/0073614 | A1 | 17 March 2016 | US entire text, all drawings WO | 2015/0080765 2016/200564 | A1 A1 | |
| US | 2021/0113155 | A1 | 22 April 2021 | WO entire text, all drawings | 2021/074292 | A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019109631 A

- JP 2011052994 A